# EUROPÄISCHE PATENTANMELDUNG

(11) **EP 0 992 487 A2**
(43) Veröffentlichungstag der Anmeldung: **12.04.2000**
(21) Anmeldenummer: 99118058.9
(22) Anmeldetag: 22.09.1999
(51) Int. Cl.: C07C 209/72

(54) **Verfahren zur Herstellung von dicycloaliphatischen Aminen**

(30) Priorität: 05.10.1998 DE 19845641
(71) Anmelder: Bayer Aktiengesellschaft, 51368 Leverkusen (DE)
(72) Erfinder: Langer, Reinhard, Dr., 47918 Tönisvorst (DE); Petruck, Gerd-Michael, Dr., 40699 Erkrath (DE)

(57) **Zusammenfassung**

Die vorliegende Erfindung betrifft ein Niederdruckverfahren zur Hydrierung von aromatischen Aminen zu den entsprechenden symmetrischen dicycloaliphatischen Aminen in Gegenwart von Rhodium-Katalysatoren die gegebenenfalls mit Ir, Ru, Os, Pd und/oder Pt oder Gemischen dieser Metalle modifiziert sind, auf mit Oxiden des Cr, Mo, W, Mn und/oder Re oder Gemischen dieser Oxide modifizierten Trägern.

## Beschreibung

Die vorliegende Erfindung betrifft ein Niederdruckverfahren zur Hydrierung von aromatischen Aminen zu den entsprechenden symmetrischen dicycloaliphatischen Aminen in Gegenwart von Rhodium-Katalysatoren die gegebenenfalls mit einem Edelmetall der Reihe Ir, Ru, Os, Pd oder Pt oder Gemischen dieser Metalle modifiziert sind, auf mit Salzen bzw. Oxiden der Metalle Cr, Mo, W, Mn und/oder Re oder Gemischen dieser Salze bzw. Oxide modifizierten Trägern.

Die katalytische Hydrierung von Anilinen zu den entsprechenden symmetrischen dicycloaliphatischen Aminen an Edelmetallkatalysatoren ist bekannt. Schriften zur Hydrierung von Anilinen an Edelmetallkontakten bei niederem Druck existieren nur wenige.

EP-A 0 324 983 und EP-A 0 324 984 beschreiben Verfahren mit basisch modifizierten Katalysatoren die sowohl Ru als auch Pd enthalten. Während in EP-A 0 324 983 unter beträchtlichem Druck gearbeitet wird und nur geringe Dicyclohexylamingehalte erzielt werden, beschreibt EP-A 0 324 984 ein Niederdruckverfahren mit hoher Dicyclohexylamin Ausbeute. Jedoch sind die Katalysatoren mit ca. 0,1-0,2 kg Edukt pro Liter Katalysator nur niedrig belastbar.

FR 1.530.477 beschreibt ein Niederdruckverfahren in dem Anilin an Pd-Trägerkatalysatoren bei Temperaturen zwischen 175 und 190°C im Wasserstoffstrom mit großen Mengen Ammoniak umgesetzt wird. Das Produkt enthält große Mengen Dicyclohexylamin.

EP-A 0 560 127 beschreibt ein Niederdruckverfahren worin Anilin an basisch modifizierten Ru-Pd-Trägerkatalysatoren umgesetzt wird. Die Katalysatoren sind nur sehr gering belastbar

In EP-A 0 208 933 werden Rh-Katalysatoren auf mit Cr-Mn-Salzen modifizierten Trägern beschrieben. Die Katalysatoren wurden zur Dehydrierung von Vorstufen zur o-Phenylphenol-Synthese bei hohen Temperaturen entwickelt.

EP-A 0 535 482 beschreibt ebenfalls temperaturresistente Rh-Katalysatoren auf mit Cr-Mn-Salzen modifizierten Trägern für die Herstellung von o-Phenylphenol, wobei die Kontakte zusätzlich zum Rh weitere Edelmetalle enthalten.

Die Rh-Katalysatoren können in thermostatisierten stationären Katalysatorschüttungen bei niedrigen Drucken und Temperaturen zwischen 300 und 400°C zur Dehydrierung eingesetzt werden.

US 5 360 934 offenbart ein Verfahren zur Hydrierung von aromatischen Aminen an einem Rhodiumkatalysator, der auf einen Träger aus κ-, θ- oder δ-Al₂O₃ aufgebracht ist. US 4 960 941 offenbart ebenfalls ein Verfahren zur Hydrierung von aromatischen Aminen an einem Rhodiumkatalysator. In diesem Fall ist der Rhodiumkatalysator auf einen Träger aus TiO₂ aufgebracht. In beiden Fällen wird die Hydrierung in flüssiger Phase unter Druck durchgeführt.

Anmeldungen zur Niederdruckhydrierung von Anilinen mit Katalysatoren die Rh als Edelmetallkomponente enthalten sind nicht bekannt, obwohl in der Literatur beschrieben wird, daß Rh-Katalysatoren für Niederdruckhydrierungen von Anilinen geeignet sein sollen (P.N. Rylander, Catalytic Hydrogenation over Platinum Metals, Academic Press 1967 S. 331-363; P.N. Rylander, Hydrogenation Methods, Academic Press 1985, S. 123-133).

Ein Vorurteil gegen die Entwicklung eines Rh-Katalysators zur Produktion von Dicyclohexylaminen bei niederem Druck erzeugte eine vor wenigen Jahren veröffentlichte Arbeit zur Gasphasen-Hydrierung von Anilin an Rh auf γ-Al₂O₃: Bei 1 atm und 200°C wurden mit steigendem Rh-Gehalt des Katalysators zwar steigende Umsätze erzielt, jedoch ist die Cyclohexylamin-Selektitivität mit ca. 20% unabhängig vom Umsatz sehr gering. Die Dicyclohexylaminselektivität sinkt sogar mit steigendem Rh-Gehalt und damit mit steigendem Umsatz von 40 auf 20%, so daß zum größten Teil unerwünschte Produkte erhalten wurden (V. Vishwanathan, S. Narayanan, J. Chem. Soc., Chem. Commun., 1990, 78-80).

Die Veröffentlichung legt nahe, daß Rhodium-Katalysatoren für technische Hydrierungen von Anilinen zu Cyclohexylaminen und Dicyclohexylaminen bei niederen Drucken in der Gasphase ungeeignet sind.

Der vorliegenden Erfindung lag die Aufgabe zugrunde, ein selektives und belastbares Niederdruckverfahren zur Hydrierung von aromatischen Aminen, bevorzugt von Anilinen zu dicycloaliphatischen Aminen, bevorzugt Dicyclohexylaminen zu finden.

Überraschenderweise wurde gefunden, daß Kontakte, die Rh auf speziell behandelten Trägermatrialien enthalten, potente Katalysatoren zur Realisierung eines Verfahrens zur Niederdruckhydrierung von aromatischen Aminen zu dicycloaliphatischen Aminen sind.

Gegenstand der Erfindung ist ein Verfahren zur Hydrierung von aromatischen Aminen zu symmetrischen dicycloaliphatischen Aminen bei Drucken zwischen 0,5 und 50 bar an mit Basen behandelten Edelmetallträgerkatalysatoren, dadurch gekennzeichnet, daß der Träger mit Salzen von Cr, Mo, W, Mn oder Re oder Gemischen dieser Salze belegt worden ist, und daß der so erhaltene Träger mit Rh als Edelmetall und gegebenenfalls einem zusätzlichen Edelmetall der Reihe Ir, Ru, Os, Pd und/oder Pt aktiviert worden ist und daß die Base durch abschließende zusätzliche Tränkung mit einer löslichen Säure ganz oder teilweise neutralisiert wird.

Geeignete Ausgangsverbindungen für das erfindungsgemäße Verfahren sind aromatische Amine wie sie beispielsweise in DE 2 502 894 und US 3 636 108 beschrieben sind. Bevorzugt sind Anilin, C₁-C₆-Alkylaniline und gegebenenfalls im Kern alkylierte C₁-C₆-alkylierte Diaminobenzole, Aminonaphthaline, C₁-C₃-alkylierte Aminonaphthaline, Diaminonaphthaline und Diamino-diphenyl-C₁-C₃-alkane.

Genannt seien beispielsweise Anilin, N-Methylanilin, N-Cyclohexylanilin, N-Cyclohexylidenanilin, o-, m-, p-Toluidin, 2,4-, 2,6-, 2,3-Diamino-toluol, Diphenylamin, 1- und 2-Aminonaphthalin, 1,4-, 1,5-, 2,5-, 2,6-, 2,7-Diaminonaphthalin und die isomeren Diaminophenylmethane.

Bevorzugt genannt seien beispielsweise Anilin, N-Cyclohexylanilin, N-Cyclohexylidenanilin, o-, m-, p-Toluidin, 2,4-, 2,6-, 2,3-Diamino-toluol, Diphenylamin.

Besonders bevorzugt genannt seien beispielsweise Anilin, 2,4- und 2,6-Diamino-toluol.

Ganz besonders bevorzugt wird das erfindungsgemäße Verfahren eingesetzt um Anilin zu hydrieren.

Die Aniline können gegebenenfalls zusammen mit zu rezyklierendem monocyclischem Amin dem Katalysator gasförmig zugeführt werden. Zur ausschließlichen Produktion von Dicyclohexylaminen wird das monocyclische Amin bevorzugt recycliert.

Die Edelmetallträgerkatalysatoren für das erfindungsgemäße Verfahren bestehen aus einem Träger, der mit einem Salz der Metalle Cr, Mo, W, Mn oder Re oder Gemischen solcher Salze belegt worden ist. Ferner beinhalten die Edelmetallträgerkatalysatoren Rh als Edelmetall und gegebenenfalls als zusätzliche Edelmetallkomponente ein Metall der Reihe Ir, Ru, Os, Pd und/oder Pt.

Geeignete Träger für die Edelmetallträgerkatalysatoren des erfindungsgemäßen Verfahrens sind Tonerden, Al₂O₃ in den verschiedenen Modifikationen (α, κ, η, γ), weiter ansonsten für Edelmetalle übliche Träger wie TiO₂, Kieselgur, Silicagel, BaCO₃, CaCO₃, ZnO, MgO, Bims, ZrO₂, Aktivkohle und die Oxide bzw. Oxidhydrate von Metallen der Reihe Cr, Mo, W, Mn und/oder Re, bevorzugt TiO₂, BaCO₃, MgO, γ-Al₂O₃ und die Oxide bzw. Oxidhydrate von Metallen der Reihe Cr, Mo, W, Mn und/oder Re, besonders bevorzugt γ- Al₂O₃, die Oxide bzw. Oxidhydrate von Metallen der Reihe Cr, Mo, W, Mn und/oder Re, ganz besonders bevorzugt γ- Al₂O₃.

Der Träger kann als Pulver oder in stückiger Form als Kugeln oder als Extrudat wie Ringe, Wagenräder u.s.w. eingesetzt werden, ferner sind Formkörper wie z.B. Wabenkörper oder Kreuzkanalstrukturen verwendbar.

Vorzugsweise wird ein Träger mit großer BET-Oberfläche eingesetzt. Die BET-Oberfläche sollte oberhalb 50 m²/g, bevorzugt zwischen 100 und 500 m²/g, besonders bevorzugt zwischen 200 und 400 m²/g liegen.

Enthält der Träger Oxide oder Oxidhydrate von Metallen der Reihe Cr, Mo, W, Mn und/oder Re oder Gemische solcher Oxide oder Oxidhydrate kann gegebenenfalls auf die nachfolgend beschriebene Modifikation des Trägers vor dem Aufbringen der Edelmetallkomponenten verzichtet werden.

Wird ein Cr, Mo, W, Mn und/oder Re-freier Träger eingesetzt, ist jener zuerst mit einer oder mehrerer dieser Komponenten zu belegen. Dies kann z.B. durch Tränken oder Besprühen des Trägers mit geeigneten Salzen dieser Elemente geschehen. Durch Trocknen und danach Tempern bei Temperaturen von ca. 200 bis 450°C werden die aufgebrachten Salze in auf dem Träger haftende Verbindungen überführt. Das Aufbringen der Verbindungen von Cr, Mo, W, Mn und/oder Re kann jedoch auch durch gemeinsames Ausfällen von Oxid-Hydroxid-Gemischen auf dem getränkten Träger mit Alkali-, Erdalkali-, oder Ammonium-Hydroxiden und gegebenenfalls anschließendes Auswaschen löslicher Anteile mit Wasser geschehen.

Besonders bevorzugt wird eine gleichmäßige Fällung durch langsame Freisetzung der Base durch Hydrolyse einer weniger basischen Vorstufe. Besonders geeignet sind dazu Hamstoffe und Urethane, ganz besonders geeignet ist Harnstoff.

Der so vorbehandelte Träger wird getrocknet und dann zwischen 10 Minuten und 10 Stunden auf 200 bis 450°C, bevorzugt 250 bis 430°C erhitzt, wobei die Temperatur innerhalb dieses Bereiches auch nach und nach erhöht werden kann.

Geeignete Salze von Cr, Mo, W, Mn und/oder Re sind beispielsweise die Acetate, Nitrate, Halogenide oder Sulfate. Ebenso geeignet sind die wasserlöslichen Oxide der höheren Oxidationsstufen der Cr, Mo, W, Mn und/oder Re-Oxide.

Bevorzugt werden Träger eingesetzt, die mit Cr- und Mn-Salzen bzw. Oxiden vorbehandelt worden sind.

Nach dem gegebenenfalls durchgeführten Auswaschen löslicher Verbindungen und der Trocknung und Temperung des mit Cr, Mo, W, Mn und/oder Re modifizierten Trägers ist der Träger zur Aufnahme der übrigen Wirkstoffe bereit.

Die übrigen Wirkstoffe sind Rh und gegebenenfalls ein Edelmetall der Reihe Ir, Ru, Os, Pd und/oder Pt, Alkali- oder Erdalkali-Hydroxid und gegebenenfalls Alkali- oder Erdalkalisulfat. Die Edelmetalle werden in Form von Lösungen ihrer Salze beispielsweise in Wasser aufgebracht. Als Salze sind beispielsweise geeignet die Halogenide, bevorzugt die Chloride, Acetate, Nitrate und Acetylacetonate. Als Alkalihydroxid sind beispielsweise NaOH oder KOH geeignet, als Erdalkalihydroxid beispielsweise Mg(OH)₂.

Als Sulfatkomponente ist beispielsweise K₂SO₄ zu nennen. Die Verbindungen können einzeln oder zusammen durch Tränken oder Besprühen aufgebracht werden. Zwischen jedem Tränkungsschritt erfolgt eine Trocknung.

Das Aufbringen von Alkali- oder Erdalkali-Hydroxid kann vor oder nach der Behandlung des Trägers mit den Edelmetallkomponenten erfolgen.

Bevorzugt wird zuerst Rh, und gegebenenfalls die Edelmetalle zur Modifizierung aufgebracht, gefolgt vom Alkalihydroxid und gegebenenfalls vom Alkalisulfat. Gegebenenfalls kann sich eine weitere Tränkung mit Base anschließen.

Nach jeder Tränkung mit Edelmetall wird gegebenenfalls mit Wasserstoff oder einem anderen Reduktionsmittel reduziert. In jedem Fall wird am Ende der letzten Trocknung z.B. mit Wasserstoff bei Temperaturen zwischen 80 und 350°C reduziert.

Nach der Reduktion der aufgebrachten Edelmetalle wird die aufdem Edelmetallträgerkatalysator befindliche Base durch Tränken mit löslichen Säuren, die in Wasser bei 25°C einen pKₐ Meiner als 5 besitzen, ganz oder teilweise neutralisiert.

Als lösliche Säuren sind geeignet, die Halogensäuren wie z.B. Salzsäure, organische Säuren wie Essigsäure, Ameisensäure oder Oxalsäure, anorganische Säuren wie z.B. Schwefelsäure oder Phoshporsäure.

Zu diesem Zweck wird zwischen 0,1 und 10 Gew.-%, bevorzugt zwischen 0,2 und 5 Gew.-% Säure auf den Edelmetallträgerkatalysator aufgebracht und der Kontakt anschließend getrocknet.

Der fertige Edelmetallträgerkatalysator enthält 0,1 bis 10 Gew.-%, bevorzugt 0,3 bis 3 Gew.-% Edelmetall der Reihe Rh, Ir, Ru, Os, Pd und/oder Pt, wobei zwischen 100 und 30 %, bevorzugt zwischen 100 und 70 % davon Rh sind. Ferner enthält der Edelmetallträgerkatalysator 0,05 bis 5 Gew.-% Cr, Mo, W, Mn und/oder Re, bevorzugt Cr und Mn. Weiterhin enthält der Edelmetallträgerkatalysator 0,05 bis 15 Gew.-% Alkali- oder Erdalkalimetallionen, 0,1 bis 10 Gew.-% Anionen saurer Verbindungen, die in Wasser gelöst bei 25°C einen pKₐ Meiner als 5 besitzen, sowie gegebenenfalls 0,05 bis 3 Gew.-% Schwefel in Form von Verbindungen, wie beispielsweise Alkalisulfate, Erdalkalisulfate, bevorzugt Kaliumsulfat.

Bevorzugt werden im erfindungsgemäßen Verfahren geeignete Edelmetallträgerkatalysatoren stückig in Form fester Schüttungen eingesetzt. Die Schüttungen können adiabat sein oder durch Verwendung von mit Wärmeträger durchströmten oder umspülten Rohrbündeln thermostatisiert werden. Ebenso ist eine Kombination aus thermostatisierten und adiabaten Schüttungen vorteilhaft, oder eine Folge von adiabaten Reaktoren mit zwischengeschalteten Kühlern. Die Ausgestaltung geeigneter Reaktoren für solche Schüttungen ist Stand der Technik und dem Fachmann bekannt.

Die Umsetzung kann in der Form erfolgen, daß beispielsweise Anilin und Wasserstoff gegebenenfalls zusammen mit zu recyclisierenden Verbindungen, wie beispielsweise Wasserstoff, Ammoniak, Cyclohexylamin erwärmt werden, die erwärmte Mischung über den Kontakt gefahren wird, ein Teil der kondensierbaren Verbindungen durch Abkühlen niedergeschlagen und zusammen mit gegebenenfalls vorher schon vorhandener Flüssigkeit ausgeschleust wird, vom verbleibenden Gasstrom ein Teil zur Ausschleusung von inerten Verbindungen abgezweigt und der Rest durch Verdichtung der Reaktion wieder zugeführt wird. Dem Reaktor wird ein gasförmiges Eduktgemisch zugeführt.

Das erfindungsgemäße Verfahren wird bei Temperaturen zwischen 50 und 250°C, bevorzugt zwischen 100 und 200°C, besonders bevorzugt zwischen 140 und 180°C durchgeführt.

Die Umsetzung erfolgt in einem Druckbereich zwischen 0,5 und 50 bar, bevorzugt zwischen 0,7 und 15 bar, besonders bevorzugt zwischen 0,9 und 8 bar.

Das umzusetzende aromatische Amin kann zusammen mit Wasserstoff im molaren Verhältnis zwischen 1/500 und 1/5, bevorzugt zwischen 1/200 und 1/10, besonders bevorzugt zwischen 1/150 und 1/40 umgesetzt werden.

Zusammen mit den aromatischen Aminen und dem Wasserstoff können geringe Mengen Ammoniak über den Kontakt gefahren werden. Ammoniak verringert die Reaktionsgeschwindigkeit deutlich und verringert die Dicyclohexylamin-Selektivität nur relativ geringfügig.

Die Belastung der Kontakte im erfindungsgemäßen Verfahren kann zwischen 0,1 und 5 kg, bevorzugt zwischen 1 und 2 kg aromatischem Amin pro Liter Katalysator und Stunde liegen.

Die Selektivitäten bezüglich dicycloaliphatischer Amine im erfindungsgemäßen Verfahren liegen deutlich über 95 %.

Das erfindungsgemäße Verfahren ermöglicht aromatische Amine in wenig aufwendigen Niederdruckapparaturen mit hoher Selektivität in dicycloaliphatische Amine umzuwandeln.

### Beispiele

### Beispiel 1 (Katalysatorpräparation)

1 L γ-Al₂O₃ der Firma Rhone-Poulenc (SPH 501, Kugeln, ⌀ = 4-6 mm, BET-Oberfläche ca. 350 m²/g) wurden mit 320 ml einer Lösung aus 30,1 g MnSO₄ × H₂O, 22,3 g (NH₄)₂Cr₂O₇ und 164 g Harnstoff getränkt. Der getränkte Träger wurde 1 h bei 90°C in einer gesättigten Wasserdampfatmosphäre bewegt. Danach erfolgten zwei Wäschen mit je 160 ml Wasser zur Entfernung löslicher Verbindungen. Der so erhaltene Träger wurde getrocknet und anschließend 30 Minuten bei 300°C in einer Drehtrommel getempert.

20,3 g RhCl₃ in 360 ml Wasser wurden durch Tränkung aufgebracht und der Katalysatorvorläufer anschließend bei 110°C getrocknet.

Danach wurden 320 ml einer Lösung aus 24 g NaOH und 24 g K₂SO₄ in Wasser aufgebracht.

Der Kontakt wurde getrocknet und 3,5 h bei 160°C im Wasserstoffstrom aktiviert.

Der Kontakt enthält 8 g Rh, 9,2 g Cr, 9,8 g Mn, 24 g NaOH und 24 g K₂SO₄ pro Liter.

Der Kontakt wurde abschließend mit einer Lösung aus 58 g H₃PO₄ in 220 g Wasser getränkt und 3 h bei 120°C im Stickstoffstrom getrocknet.

### Beispiel 2

Tabelle 1 zeigt, daß der Katalysator aus Beispiel 1 bei sehr hohen Belastungen von ca. 1,6 kg/Lxh über lange Zeiträume mit hohem Umsatz und hoher Selektivität Anilin zu Dicyclohexylamin hydriert.

Der Versuch wurde in einem ölthermostatisierten Metallrohr mit einem Durchmesser der zylinderförmigen Katalysatorschüttung von ca. 14 mm durchgeführt. Das Wasserstoff-Anilin-Gemisch wird gasförmig zugeführt.

**Tab. 1**

| Anilin-Hydrierung, 164°C Öltemperatur, 4 atm, H₂-Strom. Kat.: 50 ml Kontakt aus Beispiel 1. | | | | | | | | |
|---|---|---|---|---|---|---|---|---|
| H₂/Anilin mol/mol | Belastung kg/L×h | ANI % | CHA % | DCA % | Benzol % | LS % | UK % | Standzeit h |
| 40 | 1,64 | 0 | 39,2 | 58,1 | 2,7 | 0 | 0 | 20 |
| 40 | 1,64 | 0 | 36,1 | 61,4 | 2,5 | 0 | 0 | 145 |
| 40 | 1,64 | 0 | 38,1 | 59,9 | 2,0 | 0 | 0 | 310 |
| 40 | 1,64 | 0 | 37,7 | 60,4 | 1,9 | 0 | 0 | 480 |

Der Katalysator ist für eine technische Dicyclohexylaminproduktion besonders geeignet, weil er ferner durch Abbrennen und Reduzieren mit Wasserstoff regenerierbar ist.
ANI = Anilin, CHA = Cyclohexylamin, DCA = Dicyclohexylamin, UK = unbekannte Komponente, LS = Leichtsieder (Verbindungen, die einen niedrigeren Siedepunkt haben, als Benzol).

## Patentansprüche

1. Verfahren zur Hydrierung von aromatischen Aminen zu symmetrischen dicycloaliphatischen Aminen bei Drucken zwischen 0,5 und 50 bar an mit Basen behandelten Edelmetallträgerkatalysatoren, dadurch gekennzeichnet, daß der Träger der Edelmetallträgerkatalysatoren mit Salzen oder Oxiden von Cr, Mo, W, Mn und/oder Re oder Gemischen dieser Salze oder Oxide belegt worden ist, und daß der so erhaltene Träger mit Rh als Edelmetall und gegebenenfalls einem zusätzlichen Edelmetall der Reihe Ir, Ru, Os, Pd und/oder Pt aktiviert worden ist und daß die Base durch abschließende zusätzliche Tränkung mit einer löslichen Säure ganz oder teilweise neutralisiert wird.

2. Verfahren gemäß Anspruch 1, dadurch gekennzeichnet, daß der eingesetzte Edelmetallträgerkatalysator zu 0,1 bis 10 Gew.-% aus Edelmetall der Reihe Rh, Ir, Ru, Os, Pd und/oder Pt besteht, das zwischen 100 und 30 % Rh enthält.

3. Verfahren gemäß mindestens einem der Ansprüche 1 und 2, dadurch gekennzeichnet, daß der Edelmetallträgerkatalysator zusätzlich zum Edelmetall
0,05 bis 5 Gew.-% Cr, Mo, W, Mn und/oder Re,
0,05 bis 15 Gew.-% Alkali- oder Erdalkalimetallionen,
0,1 bis 10 Gew.-% Anionen saurer Verbindungen, die in Wasser gelöst bei 25°C einen pKₐ Meiner als 5 besitzen und gegebenenfalls
0,05 bis 3 Gew.-% Schwefel in Form von Verbindungen enthält.

4. Verfahren gemäß mindestens einem der Ansprüche 1 bis 3, dadurch gekennzeichnet, daß die Belastung der Kontakte zwischen 0,1 und 5 kg aromatischem Amin pro Liter Edelmetallträgerkatalysator und Stunde liegt.

5. Verfahren gemäß mindestens einem der Ansprüche 1 bis 4, dadurch gekennzeichnet, daß dem Reaktor eine gasförmiges Eduktgemisch zugeführt wird.
